# EUROPEAN PATENT SPECIFICATION

(11) **EP 3 323 387 B1**
(45) Date of publication and mention of the grant of the patent: **02.10.2019**
(21) Application number: 16199991.7
(22) Date of filing: 22.11.2016
(51) Int. Cl.: A61F 2/16, C08G 18/48, C08G 18/67, C08G 18/76, C08L 75/16

(54) **A BIOCOMPATIBLE COPOLYMER MATERIAL AND AN OPHTHALMIC IMPLANT CONSISTING THEREOF**
BIOKOMPATIBLES COPOLYMERMATERIAL UND EIN OPHTHALMISCHES IMPLANTAT DARAUS
MATÉRIAU COPOLYMÈRE BIOCOMPATIBLE ET IMPLANT OPHTALMIQUE EN ÉTANT CONSTITUÉ

(43) Date of publication of application: 23.05.2018
(73) Proprietor: REPER Sàrl, 1474 Luxembourg (LU)
(72) Inventor: Dzhons, Mikhael, 603018 Nizhniy Novgorod (RU); Agarkov, Viacheslav, Aliso Viejo, CA, 92656 (US)
(74) Representative: Lippert Stachow Patentanwälte Rechtsanwälte

(56) References cited:
- EP-A1- 0 584 764
- EP-A1- 1 176 454
- EP-A1- 2 436 510
- EP-A1- 2 644 348
- WO-A1-2004/031091
- WO-A1-2005/026182
- WO-A1-2005/026787
- WO-A1-2005/026826
- WO-A1-2016/115507
- JP-A- 2002 311 395
- JP-A- 2004 075 948
- RU-C1- 2 526 182
- RU-C2- 2 275 884
- US-A- 3 057 865
- US-A1- 2016 096 292
- US-B1- 6 201 036
- US-B1- 6 306 203
- ChemSpider: "3,5-DI-TERT-BUTYL-O-BENZOQUINONE", , Retrieved from the Internet: URL:http://www.chemspider.com/Chemical-Str ucture.69366.html [retrieved on 2018-03-27]
- HAMMOND D A ET AL: "Enzymatic reactions in supercritical gases", APPLIED BIOCHEMISTRY AND BIOTECHNOLOGY ; PART A: ENZYME ENGINEERING AND BIOTECHNOLOGY, HUMANA PRESS INC, NEW YORK, vol. 11, no. 5, 1 October 1985 (1985-10-01), pages 393-400, XP035176886, ISSN: 1559-0291, DOI: 10.1007/BF02798672
- TEUBER, STAIGER: "Reaktionen mit Nitrosodisulfonat, VIII. Mitteil.: ortho-Benzochinone und Phenazine", CHEMISCHE BERICHTE, vol. 88, 1955, pages 802-827,
- Jose M. Artigas ET AL: "Spectral Transmission of the Human Crystalline Lens in Adult and Elderly Persons: Color and Total Transmission of Visible Light", Investigative Opthalmology & Visual Science, vol. 53, no. 7, 25 June 2012 (2012-06-25), page 4076, XP055565246, US ISSN: 1552-5783, DOI: 10.1167/iovs.12-9471

## Description

### BACKGROUND OF THE INVENTION

Market demand of hydrophobic intraocular lenses (IOLs) increase significant in last few years and in present time more and more new researches are focused on developing new hydrophobic materials with exclusive characteristics. Interest in new material caused by improving lathe cut technology and due to significant advantages of hydrophobic IOL under hydrophilic, like biodegradation stability, low PCO rate, minimal glistening and easiest implantation technique with the use of the preload systems. There are some new advanced materials: copolymers of (meth)acrylic and silicone monomers, copolymers of modified specific (meth)acrylic monomers (for example fluorocontained (meth)acrylates) and hydrophobic polymer matrix with hydrophilic polymeric fragments. All this materials combine the useful properties of each component in the hydrophobic polymer material and allow to obtain a material with improved characteristics.

For example in US 20120309899 hydrophobic IOL material based on aryl-acrylic monomer (with its content from 30 to 60%), polydimethylsiloxane pre-polymer and addition of the acrylates with long side chain which key role is providing the optical quality of the material and increasing of hydrophobicity is described. Main advantage of this material is extremely low water absorption about 2,1 wt.%, low tack and a sufficiently high refractive index about 1,507. In other application US 20130231740 presented copolymer of hydrophobic acrylic monomer with aryl substitute and hydrophilic acrylic monomer. This combination allow to cut IOL at temperatures close to room and control the IOL power by changing the IOL hydration degree, which depends on nature of hydrophilic component and its content. However, in this case it is not clear how the material is resistant to biodegradation.

Nevertheless, all copolymers mentioned in last researches and its components may show the high hydrophobicity of final IOL, but it is not known how these materials are biocompatible. The most interesting IOL material is polymer which can combine high hydrophobicity and biocompatibility, like urethane (meth)acrylate copolymers [EP 2 644 348]. Such copolymers worked well not only in ophthalmic surgery but also in surgery [US pat. No 6201036] where show good compatibility with the tissues of the body that show extremely high biocompatibility. However, a disadvantage of IOLs made from such material is insufficient strength, uncontrolled extremely rapid opening time in the capsular bag after implantation and optic quality due to low refraction index. One more significant disadvantage is absence of any UV filter.

The nearest analog of photopolymerization composition for manufacturing the hydrophobic ophthalmologic implant based on urethane methacrylate copolymer disclosed in RU2275884 (10.07.2005, A61F2/16, A61F9/08). Above mentioned composition is a photopolymerizable mixture of:

| | |
|---|---|
| Benzyl methacrylate | 15-25 wt.% |
| Methacrylic acid | 4-8 wt.% |
| Pigment | 0.6-0.8 wt.% |
| 2,2 dimetoxy-2-phenylacetophenone | 0.1-0.8 wt.% |
| 2,4-di tert butyl-ortho-quinone | 0.001-0.006 wt.% |
| Urethane methacrylate oligomer | the rest. |

However, in this patent there is no any information about equilibrium water content at 37°C, opening time and other properties which significant effect the injection of the IOL during implantation. Furthermore, the material is not optically clear with respect to visible light so that the field of application of this material is restricted.

RU 2526182 C1 discloses a biocompatible copolymer material comprising high amounts of octyl methacrylate as (meth)acrylate monomers, not containing a polymerizable UV absorber.

US 6,201,036 B1 discloses a polymer material comprising high amounts of octyl methacrylate as (meth)acrylate monomers, not containing a polymerizable UV absorber.

US 2016/0096292 A1 and EP 2644348 A1 disclose artificial elastic implants designed for contact with body tissues like brain or intestines and related materials.

WO 2016/115507 A1 disclose polymeric compositions for manufacturing intraocular lenses comprising high amounts of methyl methacrylate.

EP 1176454 A1, EP 584764 A1, WO 2005/026826 A1, JP 2004-75948, JP 2002-311395, WO 2005/026787 A1 and WO 2005/026182 A1 disclose copolymer materials comprising urethane di(meth)acrylate oligomers and monomeric (meth)acrylates, wherein the (meth)acrylates are siloxy silyl acrylates comprising alkyl side groups like tris(trimethylsiloxy)silylpropyl acrylate.

Thus, our invention describe the biocompatible copolymer material for manufacturing hydrophobic ophthalmologic implant with improved hydrophobicity, lower glistening and optimal opening time after implantation.

### SUMMARY OF INVENTION.

The main object of this invention is to get materials combining good biocompatibility, high hydrophobicity, high optical purity, protection against UV radiation and an acceptable opening time of the final ophthalmologic implant like IOL in the capsular bag comprising the material in capsuled state before implantation.

The object is solved by a biocompatible copolymer material according to claim 1 and an ophthalmic implant, especially intraocular lens, according to claim 18. Preferred embodiments are given in the dependent claims.

The important feature is that the materials according to the present invention should be cured, preferably light-cured, which allows to obtain the non-defect and optically transparent intraocular lens with minimal water absorption and minimal glistening due to lack of microcavities and impurities inside the material. These materials we proposed contain at least one of the principal component, which is a urethane(meth)acrylate oligomer in amount 35-70% by weight, having terminal (meth)acrylate groups. The materials also contain as component b) suitable (meth)acrylic monomers in summary amount 30-65% by weight, including alkyl substituted (meth)acrylates like octyl methacrylate, aryl substituted (meth)acrylates like benzyl methacrylate, fluorinated (meth)acrylates and silicon containing (meth)acrylates to increase the refraction index, minimize stickiness and improve the optic transparency. The above-mentioned copolymer also comprises a UV-light absorber, preferably in amount of ≥ 0.2 % by weight, more preferably in amount of 0.5-5% by weight, and a polymerization initiator, preferably a photoinitiator, more preferably an UV or visible light photoinitiator like IRGACURE 651 or IRGACURE TPO, preferably in amount of 0.1-2% by weight, respectively. Furthermore, the copolymer material may include blue-light absorbing compound. The result copolymer materials are hydrophobic with equilibrium water content at 37°C less than 3%, preferably less/equal than 2.5%, low tack, high optic clearness and opening time of the final ophthalmologic implant like IOL more than 12 sec, preferably more than 15 sec. The implantable ophthalmic device like IOL produced from mentioned copolymer materials, i.e. comprising said copolymer material or consisting thereof, poses high optic performances, low tack, no glistening and controllable unfolding after injection at small incision (less than 2.4 mm) with opening time more than 14 sec, preferably 20 - 40 sec and more preferably near the 30 sec. Accordingly, the optical material of the implantable ophthalmic device like IOL comprises or consists of the claimed copolymer material.

### DETAILED DESCRIPTION OF THE INVENTION

The object of the present invention is solved by a biocompatible copolymer material according to claim 1 and by an ophthalmic implant according to claim 18.

The copolymer materials of the present invention comprises at least one main compound - urethane di(meth) acrylate oligomer, which provide high biocompatibility and hydrophobicity of the final ophthalmic device. The presented materials, however, due to their biocompatibility suitable for use not only for IOL production, but also for the corneal inlays and rings, keratoprostheses, intracapsular rings, artificial capsular bags, artificial irises and other ophthalmic devices or ophthalmic implants. The ophthalmic device or ophthalmic implant, especially the above said ones, comprise or preferably consist of the copolymer material according to the present invention.

Above mentioned urethane di(meth) acrylate oligomer (OUA) generally represents the following structure:
hydroxy(meth)acrylate' -[(diisocyanate') -(POLYOL) - (diisocyanate')] - hydroxy(meth)acrylate"
wherein hydroxy (meth)acrylate' and hydroxy (meth)acrylate" may be the same or different, diisocyanate' and diisocyanate" may be the same or different,
wherein one isocyanate group of diisocyanate' and diisocyanate", respectively, is reacted with the hydroxyl group of the hydroxy(meth)acrylate' and hydroxy(meth)acrylate", respectively under formation of an urethane group,
and wherein the other one of the isocyanate groups of diisocyanate' and diisocyanate", respectively, is reacted with a hydroxyl group of the polyol under formation of an urethane group. Especially, the hydroxy (meth)acrylate' and hydroxy (meth)acrylate" may be the same one and in combination the diisocyanate' and diisocyanate" may be the same one.

Accordingly, hydroxy (meth)acrylate moiety is the terminal group after reacting the hydroxy (meth)acrylate with the isocyanate group of the diisocyanate-polyol pre-polymer and as result the urethane (meth)acrylate oligomer can be copolymerized with (meth)acrylic monomers according to component b). These fragments may be the same or different. In general according to the present invention, the hydroxy (meth)acrylate is a (meth)acrylate ester with a polyol or preferably diol, wherein the hydroxy group of the hydroxyl (meth)acrylate is part of the ester moiety.

The presented copolymer materials comprises 35-70%, preferably 40-70% and more preferably 50-65% by weight of mentioned urethane (meth)acrylate oligomer being component a).

As the polyol can be used any branched chain diols, especially branched chain aliphatic diols, including mixtures thereof, like: polypropylene glycol (wherein polypropylene glycol preferably is applied with ethylene oxide capping in the preparation of the urethane di(meth)acrylate oligomer), dipropylene glycol, tripropylene glycol, 2-methyl-butanediol, 2-methyl-1,3-propanediol, 2,2-dimethyl-1,3 -propanediol, dibutyl-1,3 -propanediol, 2-methyl-1,3 -pentanediol, 2-ethyl-1,3-hexanediol, 1,2-octanediol and mixtures thereof. In general, the polypropylene glycol may have a molecular weight (MW) of up to 6000, preferably MW up to 2000. The used branched chain diols, especially branched chain aliphatic diols, preferably have a molecular weight MW of up to 1000, wherein they may have a molecular weight MW of up to 500 or up to 300. Preferably, the hydroxyl groups of the diol are linked to different carbon atoms, more preferably are spaced by up to 4 or up to 3 carbon atoms. Selection of polyol is determined by final elasticity of IOL, which should provide easiest implantation of IOL through small incisions. The above mentioned specific polyols are preferred to achieve improved elasticity of the copolymer material and opening time of the final ophthalmologic implant like IOL in combination with improved optical properties thereof, as mentioned above.

As isocyanate it can be used any of aromatic diisocyanates or aliphatic diisocyanates or mixtures thereof.

Aromatic diisocyanates are considered to be diisocyanates comprising an aromatic group, preferably a benzene moiety like a benzene group, toluene group, phenyl group, phenylene group etc., wherein one or both of the diisocyanate groups may be direct substituent of the aromatic group or may be spaced therefrom by preferably 1 to 3 carbon atoms, more preferably 1 or 2 carbon atoms,

Aromatic diisocyanates may be used like preferably: 2,4-toluene diisocyanate, 2,6-toluene diisocyanate, 4,4'-diphenylmethane diisocyanate, 2,4'-diphenylmethane diisocyanate, 1,3-bis(1-isocyanato-1-methylethyl)benzene, *m*-xylylene diisocyanate, *p*-tetramethyl xylene diisocyanate, 1,3-phenylene diisocyanate, 1,4-phenylene diisocyanate, 2-methyl-m-phenylene diisocyanate, 4-methyl-m-phenylene diisocyanate, 3,3'-dimethoxy-4,4'-biphenylene diisocyanate, 2,4,6-trimethyl-1,3-phenylene diisocyanate, or mixtures thereof. Preferably aromatic diisocyanates are used having one or two, preferably one, benzene moiety like benzene, toluene, phenyl, phenylene etc., as for instance the above captioned ones. Especially toluene diisocyanates like e.g. 2,4-toluene diisocyanate and 2,6-toluene diisocyanate or benzene diisocyanates like 1,3-bis(1-isocyanato-1-methylethyl)benzene are preferred. Aromatic diisocyanates are preferred to obtain improved mechanical properties like elasticity in combination with improved optic properties and low EWC (equilibrium water content) values.

Alicyclic diisocyanates may be used like: isophorone diisocyanate, cyclohexyl diisocyanate, methylcyclohexane diisocyanate, 1,3-bis(isocyanatomethyl)cyclohexane, 4,4'-methylenebis (cyclohexyl isocyanate), or mixtures thereof. Preferably, alicyclic diisocyanates comprise 1 or 2 cyclic groups, preferably 5 or 6 membered groups. Aliphatic diisocyanate may be used like hexamethylene diisocyanate, octamethylene diisocyanate, tetramethylene diisocyanate, or mixtures thereof. The aliphatic diisocyanates may comprise 2 to 16 carbon atoms or 2 to 10 carbon atoms, besides carbon atoms of isocyanate groups, respectively. Mixtures of alicyclic diisocyanates and aliphatic diisocyanates may be used.

Preferably, the amount of aromatic diisocyanates of the total amount of diisocyanates of component a) is at least 10 % by weight or more preferably at least 20 % by weight, or even at least 30 % by weight or at least 40 % by weight. The amount of aromatic diisocyanates of the total amount of diisocyanates of component a) may be at least 50 % by weight or at least 75 % by weight, or even at least 90 % by weight or about 100%. Preferably, the amount of 2,4-toluene diisocyanate or of 1,3-bis(1-isocyanato-1-methylethyl)benzene or of the combination of both of the total amount of diisocyanates of component a) is at least 10 % by weight or more preferably at least 20 % by weight, or even at least 30 % by weight or at least 40 % by weight. It has been found that a high content of aromatic diisocyanates shows improved optic and mechanic properties, especially elasticity and improved EWC values, over compositions having high amounts of aliphatic diisocyanates like isophorone diisocyanates.

As hydroxy (meth)acrylate it can be used any of 2-hydroxyethyl (meth)acrylate, 2-hydroxypropyl (meth)acrylate, 2-hydroxybutyl (meth)acrylate, di(ethylene glycol) mono-(meth)acrylate, triethylene glycol mono-(meth)acrylate, propylene glycol mono-(meth)acrylate, polyethylene glycol mono-(meth)acrylate, polypropylene glycol mono-(meth)acrylate, or mixtures thereof. In general, hydroxyalkyl (meth)acrylate (comprising a hydroxyl alkyl group) are used, wherein the alkyl group comprises up to 12 or up to 10 or preferably up to 8 carbon atoms. The alkyl group may be branched or preferably is not branched and preferably is not an alicyclic one. The term "(meth)acrylate" in general is to be understood in the meaning "acrylate" or "methacrylate" or mixtures thereof, especially "acrylate" or "methacrylate".

Component b) is present in the composition of the biocompatible copolymer material in an amount of 30-65% by weight, preferably in an amount of 35-60% by weight or more preferably in an amount of 35-55% by weight.

Component b) is selected from the group of (meth)acrylate monomers, wherein the (meth)acrylate monomers are selected from the group of alkyl (meth)acrylates, aryl (meth)acrylates, alicyclic (meth)acrylates, fluorinated (meth)acrylates, silicon containing (meth)acrylates, any of aromatic or alkyl esters of the unsaturated non (meth)acrylic carboxylic acids, and mixtures thereof,

Above mentioned copolymer include any of alkyl(meth)acrylate monomers for example tert-butyl (meth)acrylate, isobutyl (meth)acrylate, 2-ethylhexyl (meth)acrylate, octyl (meth)acrylate, isooctyl (meth)acrylate, hexyl (meth)acrylate, butyl (meth)acrylate; aryl (meth)acrylates such as 2-phenoxyethyl(meth)acrylate, benzyl methacrylate, benzyl 2-ethyl acrylate; alicyclic (meth)acrylates such as isobornyl (meth)acrylate, cyclohexyl (meth)acrylate; fluorinated (meth)acrylates such as 2,2,3,3,4,4,5,5-octafluoropentyl methacrylate, 2,2,3,3,4,4,4-heptafluorobutyl acrylate, 2,2,3,4,4,4-hexafluorobutyl methacrylate, 2,2,3,3-tetrafluoropropyl methacrylate; any of silicon containing (meth)acrylates such as trimethylsilyl (meth)acrylate, 3-(trimethoxysilyl)propyl (meth)acrylate, 3-[tris(trimethylsiloxy)silyl]propyl methacrylate. In fluorinated (meth)acrylates in general the ester group of the (meth)acrylates is fluorinated and more preferably only the ester group is fluorinated. In silicon containing (meth)acrylates preferably the ester group of the (meth)acrylates comprises silicon atoms, preferably in the form of alkyl silyl groups or alkoxysilyl groups or combinations of both.

The mentioned copolymer may also include any aromatic or alkyl esters of the unsaturated non (meth)acrylic carboxylic acids like: aromatic or alkyl esters of β-phenylacrylic acid or trans-2-butenoic acid or cis or trans butenedioic acid and others; and other polymerizable unsaturated compounds, as known to the art. The key role of mentioned co-monomers is to provide required mechanical properties, refraction index due to presence of aryl methacrylates, hydrophobicity and low water absorption of final copolymer material.

The synthesis of component a) from the said basic components is within general knowledge of a skilled person and may be achieved by any generally known standard reactions.

Component b) may comprise alkyl (meth)acrylates in an amount of ≤ 75 % by weight, preferably ≤ 50 % by weight or ≤ 35 % by weight, with respect to the total amount of component b).

Component b) may comprise alicyclic (meth)acrylates in an amount of ≤ 50 % by weight, preferably ≤ 35 % by weight or ≤ 20 % by weight or even ≤ 10 % by weight, with respect to the total amount of component b).

Component b) may comprise fluorinated (meth)acrylates in an amount of ≤ 50 % by weight, preferably ≤ 35 % by weight or ≤ 20 % by weight or even ≤ 10 % by weight, with respect to the total amount of component b).

Component b) may comprise silicon containing (meth)acrylates in an amount of ≤ 50 % by weight, preferably ≤ 35 % by weight or ≤ 20 % by weight or even ≤ 10 % by weight, with respect to the total amount of component b).

Component b) may comprise aromatic or alkyl esters of the unsaturated non (meth)acrylic carboxylic acids in an amount of ≤ 50 % by weight, preferably ≤ 35 % by weight or ≤ 20 % by weight or even ≤ 10 % by weight, with respect to the total amount of component b).

Component b) may comprise alkyl (meth)acrylates, alicyclic (meth)acrylates, fluorinated (meth)acrylates, silicon containing (meth)acrylates, any of aromatic or alkyl esters of the unsaturated non (meth)acrylic carboxylic acids in combination to a total of ≤ 50 % by weight, preferably ≤ 25 % by weight or ≤ 20 % by weight or even ≤ 10 % by weight, with respect to the total amount of component b).

The total amount of components a) and b) of the biocompatible copolymer material preferably is at least 90 wt.-%, more preferred at least 95 % by weight or at least 97 % by weight.

According to the present invention the amount of (meth)acrylic acid in the composition of the biocompatible copolymer material as a copolymer is ≤ 3 % by weight or preferably ≤ 1 % by weight or ≤ 0.5 % by weight or about 0. It has been found by the inventors that (meth)acrylic acid may deteriorate the EWC value of the biocompatible copolymer.

The weight ratio of component a) to component b) of the biocompatible copolymer material preferably is within the ratio of 2.15:1 to 1:2 or 2:1 to 1:2 or more preferably within the ratio of 1.5 : 1 to 1:1.5. Preferably the amount of component a) in the copolymer material more or less equal or higher than the amount of component b), preferably the weight ratio of component a) to component b) of the biocompatible copolymer material is within the ratio of 2:1 to 1:1.25 or is within 1.5:1 to 1:1.2.

According to the present invention the amount of (meth)acrylate monomers comprising an aromatic moiety like benzyl methacrylate, benzyl 2-ethyl acrylate of component b) is at least 50 % by weight or preferably at least 75 % by weight, or more preferably at least 80 % by weight or at least 90 % by weight. The amount of benzyl methacrylate of component b) may be at least 25 % by weight or at least 50 % by weight or even at least 75 % by weight or at least 80 % by weight. It has been shown that this is advantageous to achieve good optic and mechanical properties, including elasticity, in combination with low EWC values.

The copolymer material according to the present invention also includes an polymerizable UV absorber, to achieve required transmission in UV-Vis range and photostability, like benzophenone type absorbers such as 2-hydroxy-4-acryloyloxybenzophenone, 2-(4-Benzoyl-3-hydroxyphenoxy)ethyl acrylate and benzotriazole type polymerizable UV absorbers such as 2-[3-(2H-benzotriazol-2-yl)-4-hydroxyphenyl] ethyl methacrylate, 2- [2-hydroxy-5 - [2-(methacryloyloxy)-ethyl]phenyl]-2H-benzotriazole and other polymerizable UV absorbers as known to the art. These polymerizable UV absorbing monomers may be used also in combination of them. The UV-light absorbing component may be present in the composition of the copolymer material in amount of ≥ 0.2 % by weight, more preferably in an amount of 0.5-5% by weight or 0.75-4% by weight.

The copolymer material according to the present invention also include a polymerization initiator to achieve polymerization of the material, for instance a photoinitiator or thermal initiator, especially a photoinitiator. Any of available photoinitiators may be used such as IRGACURE 369: 2-benzyl-2-(dimethylamino)-1-[4-(4-morpholinyl)phenyl]-1-butanone; IRGACURE 651: 2,2-dimethoxy-2-phenyl acetophenone; IRGACURE TPO: 2,4,6-trimethylbenzoyldiphenylphosphine oxide; DAROCUR TPO: Diphenyl(2,4,6-trimethylbenzoyl)phosphine oxide; DAROCUR 4265: mixture of DAROCUR TPO and DAROCUR 1173: 2-Hydroxy-2-methyl-1-phenyl-1-propanone; IRGACURE 2100: mixture of 2,4,6-trimethylbenzoylphenylphosphinic acid ethyl ester and IRGACURE 819: phenyl-bis(2,4,6-trimethyl benzoyl)phosphine oxide; IRGACURE 2022: mixture of IRGACURE 819 and DAROCUR 1173; IRGACURE 2022: mixture of bis(2,6-dimethoxybenzoyl-2,4,4-trimethylpentyl)phosphine oxide and 2-hydroxy-2-methyl-1-phenyl-propane-1-one; IRGACURE 784: bis(η5-2,4-cyclopentadien-1-yl)-bis[2,6-difluoro-3-(1H-pyrrol-1-yl)phenyl]titanium; and other photoinitiators such as camphorquinone in mixture with amine accelerators like ethanol amine, diethanolamine, triethylamine, N,N-diethyl-p-tolyidine, N,N-diethyl-o-tolyidine. The polymerization initiator, especially photoinitiator or thermal initiator, preferably is present in the composition in amount of 0.1-2% by weight, or more preferably in amount of 0.2-1.5% by weight.

Preferably, the biocompatible copolymer material of the present invention is polymerized by light curing (also named: photo-curing). Light curing may be applied by irradiation of the composition with light having an appropriate wavelength, for example in the region of 330 nm to 500 nm, especially of 350 nm to 480 nm, more specifically of about 440 nm. The irradiation time might be within 2 min to 15 min, especially about 5 min. Specifically, light-curing may be applied at 440 nm for 2to 15 min, especially 5 min. The light source may be operated with a power 3-10 mW/cm². During light curing the sample is placed in a vacuum chamber. The sample to be light cured preferably is arranged between two glass plates, being transparent for the curing light. The present invention covers a respective copolymer material obtainable by light curing as described above.

Preferably, the polymerized, especially light cured, copolymer material of the present invention is extracted in isopropyl alcohol at 37°C for 24 hours and then dried in vacuum (0.1 mbar) for 12 hours at 60°C to provide a dried sample, which may be used as an implant material. Instead of isopropanol, a similar solvent having similar polarity may be used. The present invention covers a respective copolymer material obtainable by extraction and drying as described above. The extracted and dried samples are suitable to be used as an ophthalmologic implant like IOL.

The equilibrium water content (EWC) of the biocompatible copolymer material according to the present invention is determined by placed respective sample having a weight of 1g in deionized water (5 ml), heating to 70°C for 24 hours and cooling of the deionized water with sample to 37°C water temperature within 1 hour. After cooling, the sample is separated and the water content of the sample is determined as EWC value. The water content of the sample is determined by weighing of the samples and compare its weight with weight of the dry samples prior the testing, wherein drying may be achieved as described above, preferably including described isopropanol extraction.

The biocompatible copolymer material of the present invention shows an opening time of at least 14 seconds or longer, which is outstanding and very favourable in course of the implantation process. The opening time is the time of recovery of the original shape of the ophthalmologic implant after injection into capsular bag with injection system. So than larger this time it's better because the surgeon has a time to set the implant in right position without any fear to damage the capsular bag. The opening time according to the present invention is measured with a standard ophthalmologic implant, being designed as an IOL having a diameter of 12.5 mm and a double convex shape wherein when the IOL is injected into the capsular bag of the eye it has the double convex shape and 10 mm diameter.

The biocompatible copolymer material of the present invention has an refraction index n²⁰_{D} within the range of 1.4700 to 1.5200, especially within the range of 1,4750 to 1,5150.

Preferably, the biocompatible copolymer material comprises a blue-light (wavelength 430 to 490 nm) absorbing compound, in order to achieve the color of the IOL near the same as natural lense has. This compound for instance may be camphorquinone derivatives or di-alkyl-amino substituted methacrylates like: 2-(diethylamino)ethyl acrylate, 2-(diethylamino)ethyl methacrylate. This component may be present in a content from 0.2 to 2 % by weight in the composition.

Preferably, the ophthalmologic implant, especially IOL, is uniformly and of the biocompatible of the present invention, preferably as a one phase structure being made from this material.

The biocompatible copolymer material of the present invention is optically clear, that means that is does not show any opacity when visually inspected at visible light (380 nm to 780 nm) passing the copolymer material.

The invention illustrated following examples, which are not limiting the formulations for the biocompatible hydrophobic materials.

### EXAMPLE 1

The test samples from photopolymerizable compositions presented in the Table 1 were prepared as follows. Single piece IOLs (+20D) and flat samples 50x10x0.2 mm, both consisting of the biocompatible copolymer material of the present invention, were photo-cured for 10 min between two glass plates using suitable light source (Phillips UVA PL-L or Actinic BL PL-L) with power ∼ 10mW/cm², than cured in vacuum chamber with same light source for the 30 min. Cured samples were extracted in isopropyl alcohol at 37°C for 24 hours and then dried in vacuum for 12 hours at 60°C. The extracted and dried samples are suitable to be used as an ophthalmologic implant like IOL. Compositions X, Y, Z, W are not covered by the present invention.

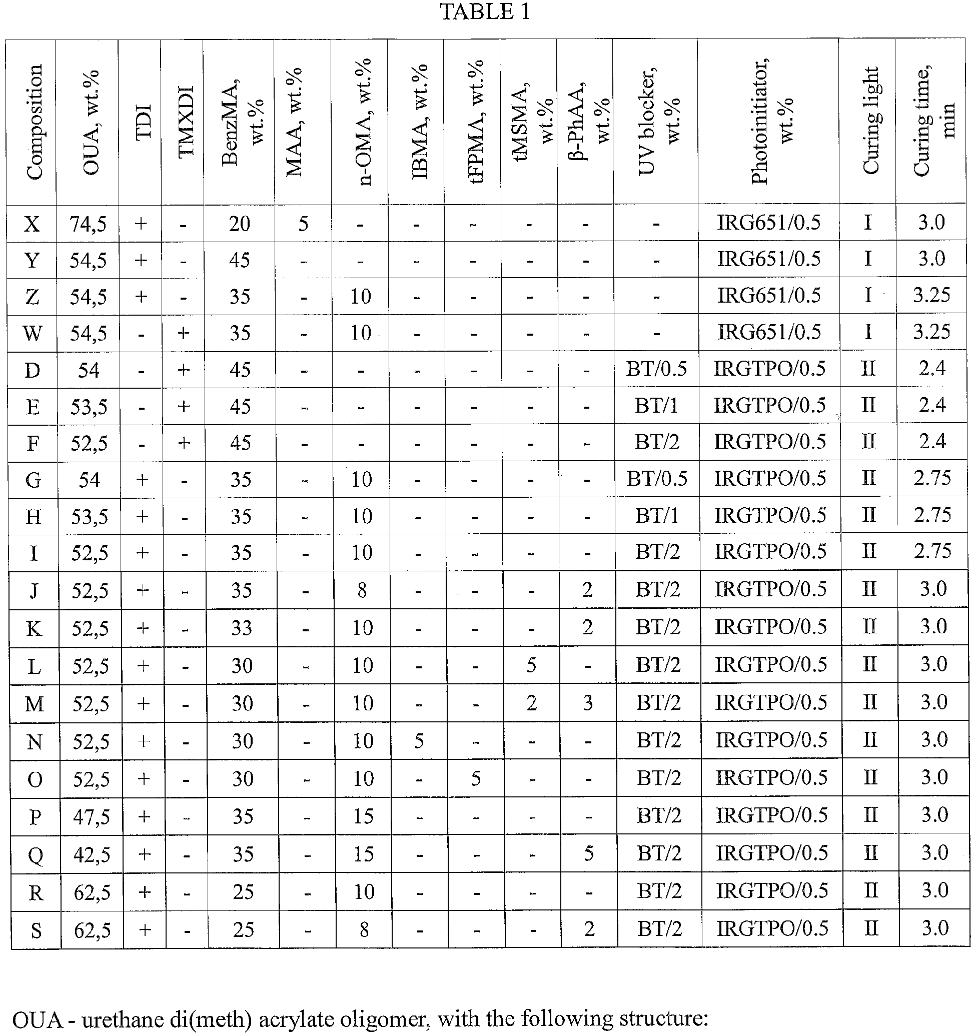

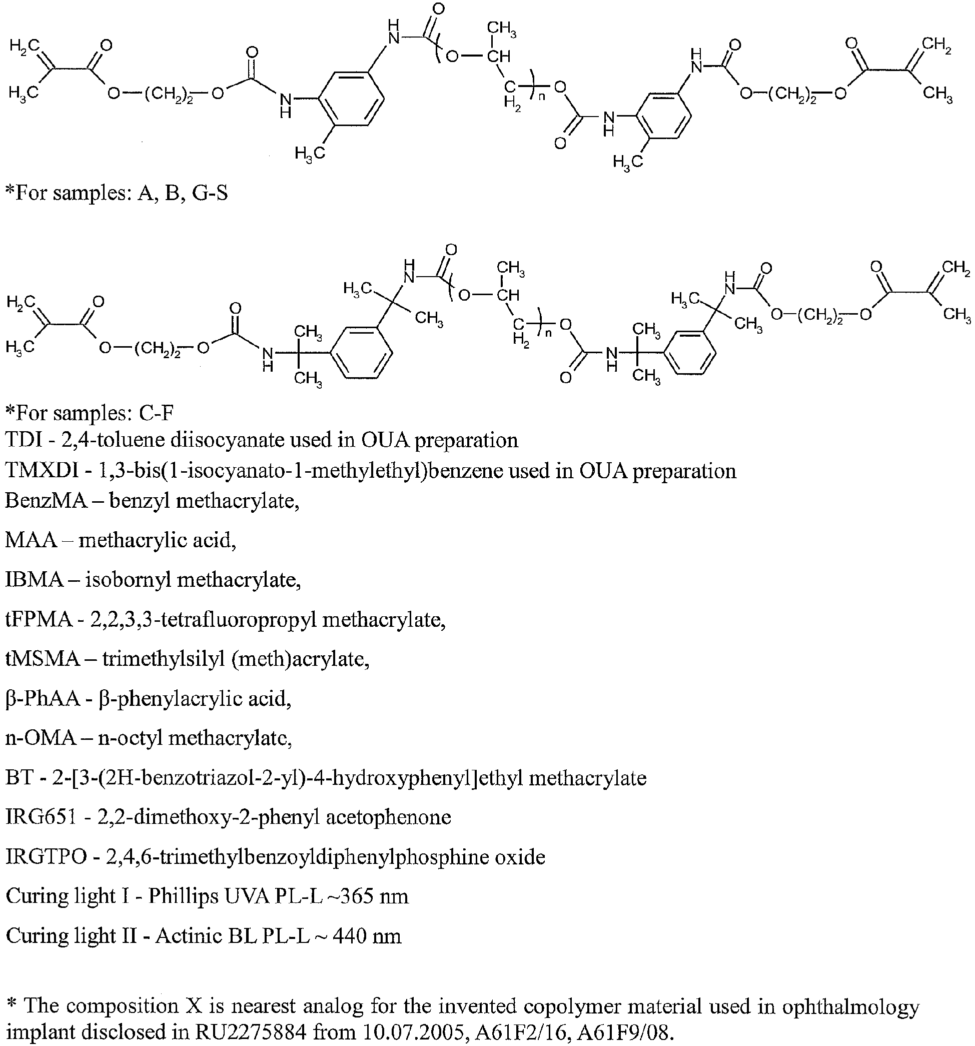

### EXAMPLE 2

After extraction according to Example 1 all samples (lg) were placed in deionized water (5ml), heated to the 70°C for 24 hours and water content after cooling of the deionized water within 1 hour to 37°C is determined as "equilibrium water content (EWC)". After cooling sample is seperated. The water content of the sample is determined by weighing of the samples and compare its weight with weight of the dry samples prior the testing.

Also visual control of IOLs after heating at 60°C for 1 hour and cooling to the 37°C was performed. IOLs were checked under microscope at 20x magnification in transmitted light and under dark field to observe level of glistening, where 1 - minimal glistening and 10 - the comparative IOL (ALCON SA60AT +20D). All results presented in Table 2. As shown in Table 2, the glistening appear mostly at water content more than 2.1 wt.% and much more lower than in comparative IOL for all compositions. Very good results are received for the sample **G, H, J, O** and **P** and even better for samples **K, M, N** and **Q** where water content are lowest in comparison to the analogs. Compositions X, Y, Z, W are not covered by the present invention.

Moreover, for this samples the opening time of final IOL also in the demand range. The opening time is the time of recovery of the original shape of the ophthalmologic implant after injection into capsular bag with injection system. So than larger this time it's better because the surgeon has a time to set the implant in right position without any fear to damage the capsular bag of the eye.

Sample **A-C** don't contain any UV blocker, so strongly decreases the possibility of practical use in IOL production or of similar implants although water content less than 3.0% and opening time is near the optimum. If compare characteristics of obtained samples with the nearest composition analog - **X** the significant difference can be observed. For all new composition lower water content, lower glistening and bigger opening time in compare with **X** can be obtained

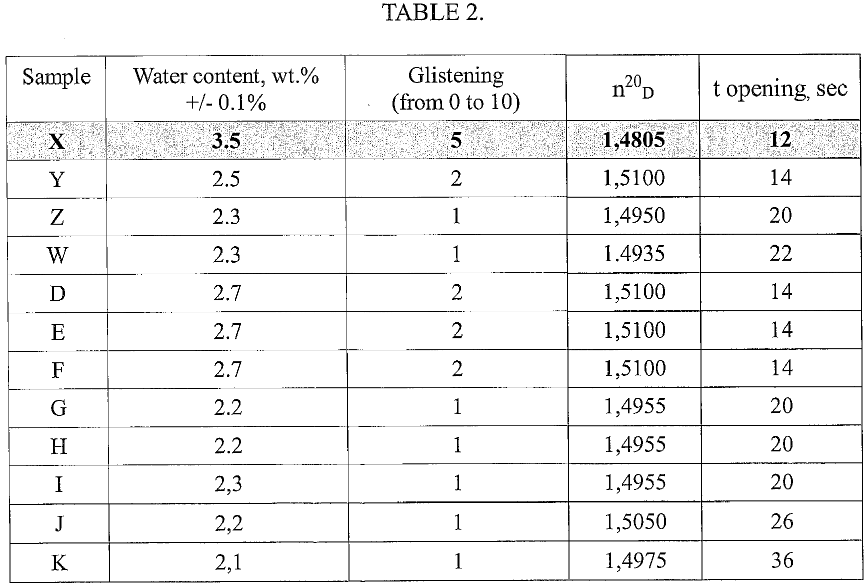

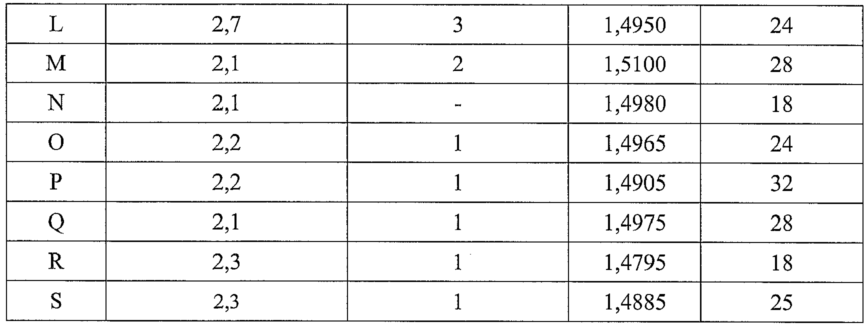

### EXAMPLE 3

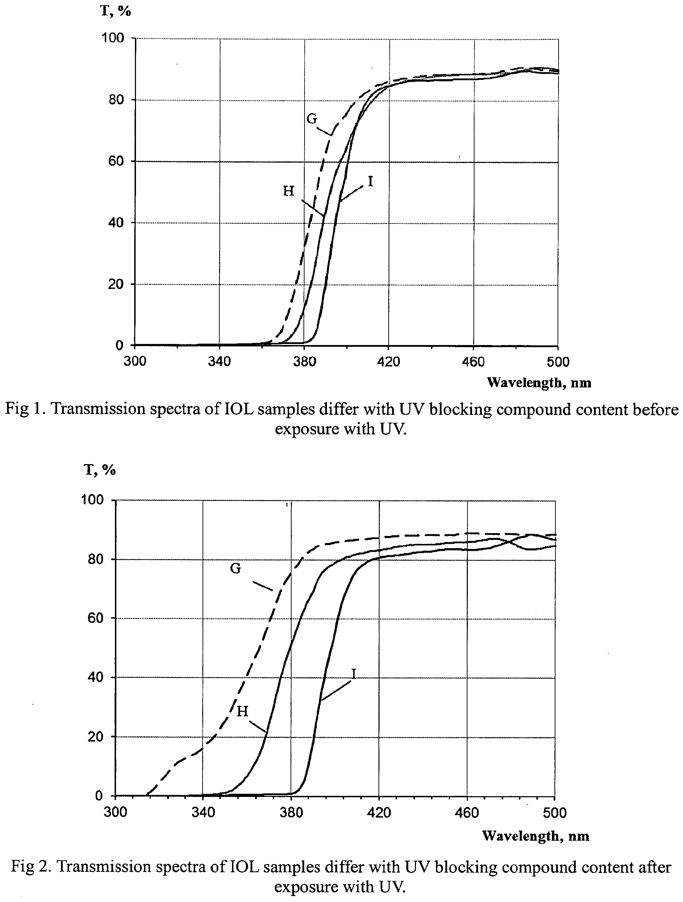

For samples G, H and I the UV spectra of IOL samples non-exposured and exposured by UV (365 nm) for 1 week and power of light source -30 mW/cm² was obtained. As shown in Fig. 1 and Fig. 2 the best spectral transmission possesses sample - **I**, (with 2 wt.% of 2-[3-(2H-benzotriazol-2-yl)-4-hydroxyphenyl] ethyl methacrylate) which have best photostability and minimal changes in transmission spectra after UV exposure. In any case, difference of sample **I** from samples **G** and **H** in amount of extractables not so significant, so it's should be optimal in comparison of all parameters. This finding also is given with respect to other UV-blockers and in combination with other compositions and other biocompatible copolymer materials according to the present invention.

This description fully presents the invention but it may be embodied in other forms without changing of its main features and embodiments mentioned above are illustrative and not limiting the invention. Moreover, any modification and combination of features in any embodiment is possible within the main scope of the invention we claimed.

## Claims

1. A biocompatible copolymer material for manufacturing optical clear hydrophobic ophthalmologic implant, formed by polymerization of a mixture comprising:
a) 35-70 wt.% of urethane di(meth)acrylate oligomer with terminal (meth)acrylate groups being prepared from the components (I) (meth)acrylate, (II) diisocyanate and (III) polyol and having the following structure:
hydroxy(meth)acrylate' - [(diisocyanate') -POLYOL- (diisocyanate")] - hydroxy(meth)acrylate",
wherein one isocyanate group of diisocyanate' and diisocyanate", respectively, is reacted with the hydroxyl group of the hydroxy(meth)acrylate' and hydroxy(meth)acrylate", respectively under formation of a urethane group,
and wherein the other one of the isocyanate groups of diisocyanate' and diisocyanate", respectively, is reacted with a hydroxyl group of the polyol under formation of a urethane group,
wherein hydroxy(meth)acrylate' and hydroxy(meth)acrylate" may be the same or different,
wherein diisocyanate' and diisocyanate" may be the same or different,
wherein the polyol in urethane di(meth)acrylate oligomer is any aliphatic branched chain diol, including mixtures thereof,
b) 30-65 wt.% of component b) being selected from the group (meth)acrylate monomers of the group of alkyl (meth)acrylates, aryl (meth)acrylates, alicyclic (meth)acrylates, fluorinated (meth)acrylates, silicon containing (meth)acrylates, any of aromatic or alkyl esters of the unsaturated non (meth)acrylic carboxylic acids and mixtures thereof, wherein the amount of (meth)acrylate monomers comprising an aromatic moiety of component b) is at least 50 % by weight,
c) not less than 0.2 wt.-% UV-light absorbing compound being a polymerizable UV-absorber, comprising benzophenone type UV-light absorbers, benzotriazole type UV-light absorbers or their combination, and
d) polymerization initiator;
wherein the mentioned biocompatible copolymer material for manufacturing hydrophobic ophthalmologic implant are hydrophobic with equilibrium water content at 37°C of less than 3.0%, wherein the amount of (meth)acrylic acid in the composition of the biocompatible copolymer material as a copolymer is ≤ 3 % by weight.

2. The biocompatible copolymer material according to claim 1, wherein the polyol in urethane di(meth)acrylate oligomer is any aliphatic branched chain diol having a MW up to 6000.

3. The biocompatible copolymer material according to claim 2, wherein the polyol in urethane di(meth)acrylate oligomer is any aliphatic branched chain diol selected from the group: polypropylene glycol having a MW up to 1000, dipropylene glycol, tripropylene glycol, 2-methyl-butanediol, 2-methyl-1,3-propanediol, 2,2-dimethyl-1,3-propanediol, dibutyl-1,3-propanediol, 2-methyl-1,3-pentanediol, 2-ethyl-1,3-hexanediol, 1,2-octanediol or mixtures thereof.

4. The biocompatible copolymer material according to any of claims 1 to 3, wherein the diisocyanate in urethane di(meth)acrylate oligomer is any of aromatic diisocyanates or aliphatic diisocyanates or mixtures thereof.

5. The biocompatible copolymer material according to claim 4, wherein the aromatic diisocyanate is selected from the group of 2,4-toluene diisocyanate, 2,6-toluene diisocyanate, 4,4'-diphenylmethane diisocyanate, 2,4'-diphenylmethane diisocyanate, 1,3-bis(1-isocyanato-1-methylethyl)benzene, m-xylylene diisocyanate, p-tetramethyl xylene diisocyanate, 1,3-phenylene diisocyanate, 1,4-phenylene diisocyanate, 2-methyl-m-phenylene diisocyanate, 4-methyl-m-phenylene diisocyanate, 3,3' -dimethoxy-4,4' -biphenylene diisocyanate, 2,4,6-trimethyl-1,3-phenylene diisocyanate or mixtures thereof.

6. The biocompatible copolymer material according to claim 4 or 5, wherein the aliphatic diisocyanate is selected from the group of isophorone diisocyanate, cyclohexyl diisocyanate, methylcyclohexane diisocyanate, 1,3-bis(isocyanatomethyl)cyclohexane,4,4'-methylenebis (cyclohexyl isocyanate), hexamethylene diisocyanate, octamethylene diisocyanate, tetramethylene diisocyanate or mixtures thereof.

7. The biocompatible copolymer material according to any of claims 1 to 6 ,wherein the hydroxy (meth)acrylate of the urethane di(meth)acrylate oligomer is a hydroxy (meth)acrylate selected from the group: 2-hydroxyethyl (meth)acrylate, 2-hydroxypropyl (meth)acrylate, 2-hydroxybutyl (methacrylate), di(ethylene glycol) mono-(meth)acrylate, triethylene glycol mono-(meth)acrylate, propylene glycol mono-(meth)acrylate, polyethylene glycol mono-(meth)acrylate, polypropylene glycol mono-(meth)acrylate or mixtures thereof.

8. The biocompatible copolymer material according to any of claims 1 to 7, wherein the alkyl (meth)acrylate monomers of component b) is selected from the group: tert-butyl (meth)acrylate, isobutyl (meth)acrylate, 2-ethylhexyl (meth)acrylate, octyl (meth)acrylate, isooctyl (meth)acrylate, hexyl (meth)acrylate, butyl (meth)acrylate or mixtures thereof.

9. The biocompatible copolymer material according to any of claims 1 to 8, wherein the aryl(meth)acrylate monomers of component b) is selected from the group: 2-phenoxyethyl(meth)acrylate, benzyl methacrylate and benzyl 2-ethyl acrylate or mixtures thereof.

10. The biocompatible copolymer material according to any of claims 1 to 9, wherein the alicyclic (meth)acrylate monomers of component b) is selected from the group: isobornyl (meth)acrylate and cyclohexyl (meth)acrylate or mixtures thereof.

11. The biocompatible copolymer material according to any of claims 1 to 10, wherein the fluorinated (meth)acrylate monomers of component b) selected from the group: 2,2,3,3,4,4,5,5-octafluoropentyl methacrylate, 2,2,3,3,4,4,4-heptafluorobutyl acrylate, 2,2,3,4,4,4-hexafluorobutyl methacrylate, 2,2,3,3-tetrafluoropropyl methacrylate or mixtures thereof.

12. The biocompatible copolymer material according to any of claims 1 to 11, wherein the silicon containing (meth)acrylate monomers of component b) selected from the group: trimethylsilyl(meth)acrylate, 3-(trimethoxysilyl)propyl (meth)acrylate, 3-[tris(trimethylsiloxy) silyl]propyl methacrylate or mixtures thereof.

13. The biocompatible copolymer material of any of claims 1 to 12, wherein the total of components a) and b) of the biocompatible copolymer material is at least 90 wt.-%.

14. The biocompatible copolymer material of any of claims 1 to 13, wherein the weight ratio of components a) and b) of the biocompatible copolymer material is within the ratio of 2:1 to 1:2.

15. The biocompatible copolymer material of any of claims 1 to 14, wherein the content of benzyl methacrylate in the biocompatible copolymer material is at least 25 wt.-%.

16. The biocompatible copolymer material of any of claims 1 to 15, wherein the UV-absorbing compound is present in the biocompatible material in a content of 0.5 to 5 wt.-%.

17. The biocompatible copolymer material according to any of claims 1 to 16, wherein the polymerizing mixture comprises blue-light (wavelength 430 to 490 nm) absorbing compound.

18. Ophthalmic implant, wherein the material of the ophthalmic implant is comprising or consisting of a copolymer material according to any of claims 1 to 17.

## Patentansprüche

1. Biokompatibles Copolymermaterial zur Herstellung optisch klarer hydrophober ophthalmologischer Implantate, gebildet durch Polymerisation einer Mischung enthaltend:
a) 35-70 Gewichtsprozent Urethandi(meth)acrylatoligomer mit terminalen (Meth)acrylatgruppen, hergestellt aus den Komponenten (I) (Meth)acrylat, (II) Diisocyanat und (III) Polyol, mit der folgenden Struktur
Hydroxy(meth)acrylat'-[(Diisocyanat')-Polyol-(Diisocyanat")]-Hydroxy(meth)acrylat",
wobei jeweils eine Isocyanatgruppe aus Diisocyanat' bzw. Diisocyanat" mit jeweils der Hydroxylgruppe des Hydroxy(meth)acrylat' bzw. Hydroxy(meth)acrylat" unter Bildung einer Urethangruppe umgesetzt wird,
und wobei die jeweils andere Isocyanatgruppe aus Diisocyanat' bzw. Diisocyanat' mit jeweils einer Hydroxylgruppe des Polyols unter Bildung einer Urethangruppe umgesetzt wird, wobei Hydroxy(meth)acrylat' und Hydroxy(meth)acrylat" gleich oder verschieden sein können,
wobei Diisocyanat' und Diisocyanat" gleich oder verschieden sein können,
wobei der Polyol im Urethandi(meth)acrylatoligomer ein beliebiger aliphatischer verzweigtes Diol unter Einschluss von deren Mischungen ist,
b) 30-65 Gewichtsprozent einer Komponente b), ausgewählt aus der aus (Meth)acrylatmonomere der Gruppe Alkyl(meth)acrylate, Aryl(meth)acrylate, alizyklische (Meth)acrylate, fluorierte (Meth)acrylate, siliziumhaltige (Meth)acrylate, beliebige aromatische oder Alkylester von ungesättigten nicht-(Meth)acrylsäuren und Mischungen derselben, wobei die Menge der (Meth)acrylatmonomere mit einer aromatischen Gruppe der Komponente b) mindestens 50 Gewichtsprozent beträgt,
c) nicht weniger als 0,2 Gewichtsprozent einer UV-Licht absorbieren Verbindung, die ein polymerisierbarer UV-Absorber, umfassend UV-Lichtabsorber vom Typ Benzophenon, UV-Lichtabsorber vom Typ Benztriazol oder deren Kombination, ist und
d) ein Polymerisationsinitiator,
wobei das genannte biokompatible Copolymermaterial zur Herstellung hydrophober ophthalmologischer Implantate hydrophob mit einem Gleichgewichtswassergehalt bei 37 °C von weniger als 3,0 % ist,
wobei die Menge von (Meth)acrylsäure in der Zusammensetzung des biokompatiblen Copolymermaterials als Copolymer ≤ 3 Gewichtsprozent beträgt.

2. Biokompatibles Copolymermaterial nach Anspruch 1, wobei das Polyol im Urethandi(meth)acrylatoligomer ein beliebiges aliphatisches verzweigtes Diol mit einem MW bis zu 6000 ist.

3. Biokompatibles Copolymermaterial nach Anspruch 2, wobei das Polyol im Urethandi(meth)acrylatoligomer ein beliebiges aliphatisches verzweigtes Diol, ausgewählt aus der Gruppe Polypropylenglykol mit MW bis zu 1000, Dipropylenglykol, Tripropylenglykol, 2-Methylbutandiol, 2-Methyl-1,3-propandiol, 2,2-Dimethyl-1,3-propandiol, Dibutyl-1,3-propandiol, 2-Methyl-1,3-pentandiol, 2-Ethyl-1,3-hexandiol, 1,2-Oktandiol oder Mischungen aus diesen, ist.

4. Biokompatibles Copolymermaterial nach einem der Ansprüche 1 bis 3, wobei das Diisocyanat im Urethandi(meth)acrylatoligomer ein beliebiges aromatisches Diisocyanat oder aliphatisches Diisocyanat oder Mischungen daraus ist.

5. Biokompatibles Copolymermaterial nach Anspruch 4, wobei das aromatische Diisocyanat ausgewählt ist aus der Gruppe 2,4-Toluoldiisocyanat, 2,6-Toluoldiisocyanat, 4,4'-Diphenylmethandiisocyanat, 2,4'-Diphenylmethandiisocyanat, 1,3-Bis(1-isocyanato-1-methylethyl)benzol, m-Xylylendiisocyanat, p-Tetramethylxyloldiisocyanat, 1,3-Phenylendiisocyanat, 1,4-Phenylendiisocyanat, 2-Methyl-m-phenylendiisocyanat, 4-Methyl-m-phenylendiisocyanat, 3,3'-Dimethoxy-4,4'-biphenylendiisocyanat, 2,4,6-Trimethyl-1,3-phenylendiisocyanat oder Mischungen aus diesen.

6. Biokompatibles Copolymermaterial nach Anspruch 4 oder 5, wobei das aliphatische Diisocyanat ausgewählt ist aus der Gruppe Isophorondiisocyanat, Cyclohexyldiisocyanat, Methylcyclohexandiisocyanat, 1,3-bis(Isocyanatomethyl)cyclohexan, 4,4'-Methylen-bis-(cyclohexylisocyanat), Hexamethylendiisocyanat, Octamethylendiisocyanat, Tetramethylendiisocyanat oder Mischungen aus diesen.

7. Biokompatibles Copolymermaterial nach einem der Ansprüche 1 bis 6, wobei das Hydroxy(meth)acrylat des Urethandi(meth)acrylatoligomers ein aus der Gruppe 2-Hydroxyethyl(meth)acrylat, 2-Hydroxypropyl(meth)acrylat, 2-Hydroxybutyl(meth)acrylat, Di(ethylenglykol)-mono(meth)acrylat, Triethylenglykol-mono-(meth)acrylate, Propylen glykol mono-(meth)acrylate, Polyethylen glykol mono-(meth)acrylate, Polypropylenglykolmono-(meth)acrylat oder Mischungen aus diesen ausgewähltes Hydroxy(meth)acrylat ist.

8. Biokompatibles Copolymermaterial nach einem der Ansprüche 1 bis 7, wobei die Alkyl(meth)acrylatmonomere des Bestandteils b) ausgewählt sind aus der Gruppe t-Butyl(meth)acrylat, i-Butyl(meth)acrylat, 2-Ethylhexyl(meth)acrylat, Octyl(meth)acrylat, i-Octyl(meth)acrylat, Hexyl(meth)acrylat, Butyl(meth)acrylat und Mischungen aus diesen.

9. Biokompatibles Copolymermaterial nach einem der Ansprüche 1 bis 8, wobei die Aryl(meth)acrylatmonomere des Bestandteils b) ausgewählt sind aus der Gruppe 2-Phenoxyethyl(meth)acrylat, Benzylmethacrylat und Benzyl-2-ethylacrylat oder Mischungen aus diesen.

10. Biokompatibles Copolymermaterial nach einem der Ansprüche 1 bis 9, wobei die alizyklischen (Meth)acrylatmonomere des Bestandteils b) ausgewählt sind aus der Gruppe Isobornyl(meth)acrylat und Cyclohexyl(meth)acrylat oder Mischungen aus diesen.

11. Biokompatibles Copolymermaterial nach einem der Ansprüche 1 bis 10, wobei die florierten (Meth)acrylatmonomere des Bestandteils b) ausgewählt sind aus der Gruppe 2,2,3,3,4,4,5,5-Octafluorpentylmethacrylat, 2,2,3,3,4,4,4-Heptafluorbutylacrylat, 2,2,3,4,4,4-Hexafluorbutylmethacrylat, 2,2,3,3-Tetrafluorpropylmethacrylat oder Mischungen aus diesen.

12. Biokompatibles Copolymermaterial nach einem der Ansprüche 1 bis 11, wobei die siliziumhaltigen (Meth)acrylatmonomere des Bestandteils b) ausgewählt sind aus der Gruppe Trimethylsilyl(meth)acrylat, 3-(Trimethoxysilyl)propyl(meth)acrylat, 3-[Tris(trimethylsiloxy)silyl]propylmethacrylat oder Mischungen aus diesen.

13. Biokompatibles Copolymermaterial nach einem der Ansprüche 1 bis 12, wobei der Gesamtanteil der Bestandteile a) und b) des biokompatiblen Copolymermaterials mindestens 90 Gewichtsprozent beträgt.

14. Biokompatibles Copolymermaterial nach einem der Ansprüche 1 bis 13, wobei das Gewichtsverhältnis der Bestandteile a) und b) des biokompatiblen Copolymermaterials innerhalb des Verhältnisses 2:1 bis 1:2 liegt.

15. Biokompatibles Copolymermaterial nach einem der Ansprüche 1 bis 14, wobei der Gehalt an Benzylmethacrylat im biokompatiblen Copolymermaterial mindestens 25 Gewichtsprozent beträgt.

16. Biokompatibles Polymermaterial nach einem der Ansprüche 1 bis 15, wobei die UVabsorbierende Verbindung im biokompatiblen Material mit einem Gehalt von 0,5 bis 5 Gewichtsprozent vorliegt.

17. Biokompatibles Copolymermaterial nach einem der Ansprüche 1 bis 16, wobei das Polymerisationsgemisch eine Verbindung enthält, die blaues Licht (Wellenlänge 430 bis 490 nm) absorbiert.

18. Ophthalmologisches Implantat, wobei das Material des ophthalmologischen Implantats ein Copolymermaterial nach einem der Ansprüche 1 bis 17 umfasst oder daraus besteht.

## Revendications

1. Matériau copolymère biocompatible destiné à fabriquer un implant ophtalmologique hydrophobe clair optique, formé par polymérisation d'un mélange comprenant :
a) 35 à 70 % en poids d'un oligomère de di(méth)acrylate d'uréthane avec des groupes (méth)acrylate terminaux qui est préparé à partir des composants (I) (méth)acrylate, (II) diisocyanate et (III) polyol et ayant la structure suivante :
hydroxy(méth)acrylate' - [(diisocyanate')-POLYOL-(diisocyanate'')]-hydroxy(méth)acrylate'',
dans lequel un groupe isocyanate de diisocyanate' et diisocyanate'', respectivement, est mis à réagir avec le groupe hydroxyle de l'hydroxy(méth)acrylate' et l'hydroxy(méth)acrylate'', respectivement sous formation d'un groupe uréthane,
et dans lequel l'autre des groupes isocyanate de diisocyanate' et diisocyanate'', respectivement, est mis à réagir avec un groupe hydroxyle du polyol sous formation d'un groupe uréthane,
dans lequel l'hydroxy(méth)acrylate' et l'hydroxy(méth)acrylate'' peuvent être identiques ou différents, dans lequel le diisocyanate' et le diisocyanate'' peuvent être identiques ou différents,
dans lequel le polyol dans l'oligomère de di(méth)acrylate d'uréthane est tout diol à chaîne ramifiée aliphatique, dont ses mélanges,
b) 30 à 65 % en poids de composant b) étant choisis dans le groupe des monomères de (méth)acrylate du groupe des (méth)acrylates d'alkyle, (méth)acrylates d'aryle, (méth)acrylates alicycliques, (méth)acrylates fluorés, (méth)acrylates contenant du silicium, l'un quelconque d'esters aromatiques ou d'alkyle des acides carboxyliques non (méth)acryliques insaturés et de leurs mélanges, dans lequel la quantité de monomères de (méth)acrylate comprenant une fraction aromatique de composant b) est d'au moins 50 % en poids,
c) pas moins de 0,2 % en poids de composé absorbant la lumière UV qui est un absorbeur UV polymérisable, comprenant des absorbeurs de lumière UV de type benzophénone, des absorbeurs de lumière UV de type benzotriazole, ou leur combinaison, et
d) un initiateur de polymérisation ;
dans lequel le matériau copolymère biocompatible mentionné destiné à fabriquer un implant ophtalmologique hydrophobe est hydrophobe avec une teneur en eau à l'équilibre à 37 °C de moins de 3,0 %, dans lequel la quantité d'acide (méth)acrylique dans la composition du matériau copolymère biocompatible en tant que copolymère est ≤ 3 % en poids.

2. Matériau copolymère biocompatible selon la revendication 1, dans lequel le polyol dans l'oligomère de di(méth)acrylate d'uréthane est tout diol à chaîne ramifiée aliphatique ayant une MM (masse moléculaire) allant jusqu'à 6 000.

3. Matériau copolymère biocompatible selon la revendication 2, dans lequel le polyol dans l'oligomère de di(méth)acrylate d'uréthane est tout diol à chaîne ramifiée aliphatique choisi dans le groupe : un polypropylène glycol ayant une MM allant jusqu'à 1 000, le dipropylène glycol, le tripropylène glycol, le 2-méthyl-butanediol, le 2-méthyl-1,3-propanediol, le 2,2-diméthyl-1,3-propanediol, le dibutyl-1,3-propanediol, le 2-méthyl-1,3-pentanediol, le 2-éthyl-1,3-hexanediol, le 1,2-octanediol ou leurs mélanges.

4. Matériau copolymère biocompatible selon l'une quelconque des revendications 1 à 3, dans lequel le diisocyanate dans l'oligomère de di(méth)acrylate d'uréthane est l'un quelconque de diisocyanates aromatiques ou de diisocyanates aliphatiques ou leurs mélanges.

5. Matériau copolymère biocompatible selon la revendication 4, dans lequel le diisocyanate aromatique est choisi dans le groupe du diisocyanate de 2,4-toluène, du diisocyanate de 2,6-toluène, du diisocyanate de 4,4'-diphénylméthane, du diisocyanate de 2,4'-diphénylméthane, du 1,3-bis(1-isocyanato-1-méthyléthyl)benzène, du diisocyanate de m-xylylène, du diisocyanate de p-tétraméthyl xylène, du diisocyanate de 1,3-phénylène, du diisocyanate de 1,4-phénylène, du diisocyanate de 2-méthyl-m-phénylène, du diisocyanate de 4-méthyl-m-phénylène, du diisocyanate de 3,3'-diméthoxy-4,4'-biphénylène, du diisocyanate de 2,4,6-triméthyl-1,3-phénylène ou de leurs mélanges.

6. Matériau copolymère biocompatible selon la revendication 4 ou 5, dans lequel le diisocyanate aliphatique est choisi dans le groupe du diisocyanate d'isophorone, du diisocyanate de cyclohexyle, du diisocyanate de méthylcyclohexane, du 4,4'-méthylènebis(cyclohexyl isocyanate) de 1,3-bis(isocyanatométhyl)cyclohexane, du diisocyanate d'hexaméthylène, du diisocyanate d'octaméthylène, du diisocyanate de tétraméthylène ou leurs mélanges.

7. Matériau copolymère biocompatible selon l'une quelconque des revendications 1 à 6, dans lequel l'hydroxy(méth)acrylate de l'oligomère de di(méth)acrylate d'uréthane est un hydroxy(méth)acrylate choisi dans le groupe : le (méth)acrylate de 2-hydroxyéthyle, le (méth)acrylate de 2-hydroxypropyle, le (méth)acrylate de 2-hydroxybutyle, le mono-(méth)acrylate de di(éthylène glycol), le mono-(méth)acrylate de triéthylène glycol, le mono-(méth)acrylate de propylène glycol, le mono-(méth)acrylate de polyéthylène glycol, le mono-(méth)acrylate de polypropylène glycol ou leurs mélanges.

8. Matériau copolymère biocompatible selon l'une quelconque des revendications 1 à 7, dans lequel les monomères de (méth)acrylate d'alkyle du composant b) sont choisis dans le groupe : le (méth)acrylate de tert-butyle, le (méth)acrylate d'isobutyle, le (méth)acrylate de 2-éthylhexyle, le (méth)acrylate d'octyle, le (méth)acrylate d'isooctyle, le (méth)acrylate d'hexyle, le (méth)acrylate de butyle ou leurs mélanges.

9. Matériau copolymère biocompatible selon l'une quelconque des revendications 1 à 8, dans lequel les monomères d'aryl(méth)acrylate du composant b) sont choisis dans le groupe : le (méth)acrylate de 2-phénoxyéthyle, le (méth)acrylate de benzyle et l'acrylate de benzyl 2-éthyle ou leurs mélanges.

10. Matériau copolymère biocompatible selon l'une quelconque des revendications 1 à 9, dans lequel les monomères de (méth)acrylate alicyclique du composant b) sont choisis dans le groupe : le (méth)acrylate d'isobornyle et le (méth)acrylate de cyclohexyle ou leurs mélanges.

11. Matériau copolymère biocompatible selon l'une quelconque des revendications 1 à 10, dans lequel les monomères de (méth)acrylate fluorés du composant b) sont choisis dans le groupe : le méthacrylate de 2,2,3,3,4,4,5,5-octafluoropentyle, l'acrylate de 2,2,3,3,4,4,4-heptafluorobutyle, le méthacrylate de 2,2,3,4,4,4-hexafluorobutyle, le méthacrylate de 2,2,3,3-tétrafluoropropyle ou leurs mélanges.

12. Matériau copolymère biocompatible selon l'une quelconque des revendications 1 à 11, dans lequel les monomères de (méth)acrylate contenant du silicium du composant b) sont choisis dans le groupe : le (méth)acrylate de triméthylsilyle, le (méth)acrylate de 3-(triméthoxysilyl)propyle, le méthacrylate de 3-[tris(triméthylsiloxy)silyl]propyle ou leurs mélanges.

13. Matériau copolymère biocompatible selon l'une quelconque des revendications 1 à 12, dans lequel le total des composants a) et b) du matériau copolymère biocompatible est d'au moins 90 % en poids.

14. Matériau copolymère biocompatible selon l'une quelconque des revendications 1 à 13, dans lequel le rapport en poids des composants a) et b) du matériau copolymère biocompatible entre dans le rapport de 2:1 à 1:2.

15. Matériau copolymère biocompatible selon l'une quelconque des revendications 1 à 14, dans lequel la teneur en méthacrylate de benzyle dans le matériau copolymère biocompatible est d'au moins 25 % en poids.

16. Matériau copolymère biocompatible selon l'une quelconque des revendications 1 à 15, dans lequel le composé absorbant les UV est présent dans le matériau biocompatible dans une teneur de 0,5 à 5 % en poids.

17. Matériau copolymère biocompatible selon l'une quelconque des revendications 1 à 16, dans lequel le mélange de polymérisation comprend un composant absorbant la lumière bleue (longueur d'onde 430 à 490 nm).

18. Implant ophtalmique, dans lequel le matériau de l'implant ophtalmique comprend ou consiste en un matériau copolymère selon l'une quelconque des revendications 1 à 17.
